# EUROPÄISCHE PATENTANMELDUNG

(11) **EP 2 974 758 A1**
(43) Veröffentlichungstag der Anmeldung: **20.01.2016**
(21) Anmeldenummer: 14177069.3
(22) Anmeldetag: 15.07.2014
(51) Int. Cl.: A61M 5/20, A61M 5/32

(54) **Zylinder-Kolben-Einheit mit Kanülen**

(71) Anmelder: LTS LOHMANN Therapie-Systeme AG, 56626 Andernach (DE)
(72) Erfinder: Hoffmann, Hans-Rainer, 56566 Neuwied (DE)

(57) **Zusammenfassung**

Die Erfindung betrifft eine Zylinder-Kolben-Einheit (10) mit einem einen Zylinderinnenraum begrenzenden Zylinder (11) und mit einem in diesem geführten Kolben, wobei der Zylinder einen Zylinderboden mit mindestens einem Durchbruch (15) aufweist. Im Durchbruch ist eine Kanüle (50) verschiebbar gelagert. Die Kanüle ist an einem im Zylinderinnenraum angeordneten, vom Kolben getrennten Vorschubkolben (61) befestigt.

Mit der vorliegenden Erfindung wird eine Vorrichtung entwickelt, die ein schmerzarmes Einbringen der Injektionslösung ermöglicht und die Gefahr von Verletzungen reduziert.

## Beschreibung

Die Erfindung betrifft eine Zylinder-Kolben-Einheit mit einem einen Zylinderinnenraum begrenzenden Zylinder und mit einem in diesem geführten Kolben, wobei der Zylinder einen Zylinderboden mit mindestens einem Durchbruch aufweist.

Um eine Injektionslösung in die Haut eines Patienten einzubringen, muss die Injektionslösung die Hornhaut passieren. Hierzu wird im Allgemeinen eine Nadel oder ein hoher Druck eingesetzt. Dies kann beim Patienten Schmerzen erzeugen. Außerdem besteht für den Anwender Verletzungsgefahr.

Der vorliegenden Erfindung liegt die Problemstellung zugrunde, eine Vorrichtung zu entwickeln, die ein schmerzarmes Einbringen der Injektionslösung ermöglicht und die Gefahr von Verletzungen reduziert.

Diese Problemstellung wird mit den Merkmalen des Hauptanspruchs gelöst. Dazu ist im Durchbruch eine Kanüle verschiebbar gelagert. Die Kanüle ist an einem im Zylinderinnenraum angeordneten, vom Kolben getrennten Vorschubkolben befestigt.

Weitere Einzelheiten der Erfindung ergeben sich aus den Unteransprüchen und den nachfolgenden Beschreibungen schematisch dargestellter Ausführungsbeispiele.
- Figur 1:: Zylinder-Kolben-Einheit vor dem Injizieren;
- Figur 2:: Zylinder-Kolben-Einheit aus Figur 1 beim Injizieren;
- Figur 3:: Zylinder-Kolben-Einheit mit membranartigem Vorschubkolben vor dem Injizieren;
- Figur 4:: Zylinder-Kolben-Einheit aus Figur 3 beim Injizieren;
- Figur 5:: Zylinder-Kolben-Einheit mit Druckfeder als Rückstellelement;
- Figur 6:: Zylinder-Kolben-Einheit mit Biegefeder als Rückstellelement;
- Figur 7:: Draufsicht der Biegefeder aus Figur 6;
- Figur 8:: Variante der Biegefeder aus Figur 6;
- Figur 9:: Zylinder-Kolben-Einheit mit konvexer Auflagefläche;
- Figur 10:: Zylinder-Kolben-Einheit aus Figur 9 beim Injizieren;
- Figur 11:: Zylinder-Kolben-Einheit mit in Hubzylindern geführten Vorschubkolben.

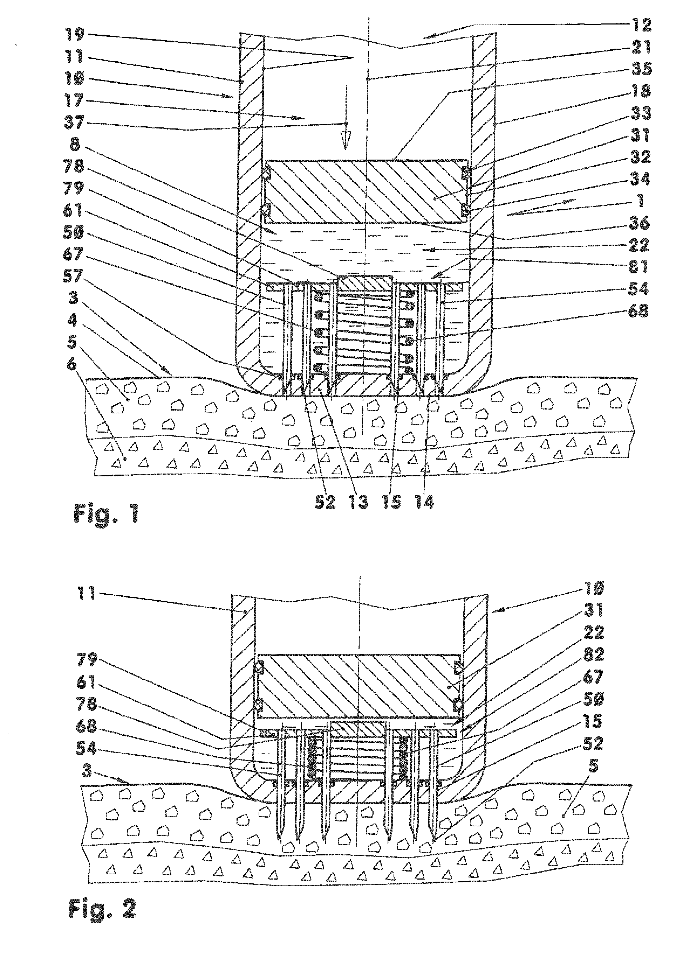

Die Figuren 1 und 2 zeigen in Schnittdarstellungen eine Zylinder-Kolben-Einheit (10) einer Injektionsvorrichtung. Mittels der Injektionsvorrichtung werden beispielsweise In-jektionslösungen (8) in die Haut (3) eines Patienten eingebracht. Die z.B. wirkstoffhaltige Injektionslösung (8) gelangt hierbei in die unterhalb der Hornhaut (4) liegenden Hautschichten (5, 6).

Die Zylinder-Kolben-Einheit (10) umfasst einen topfartig ausgebildeten Zylinder (11) und einen im Zylinder (11) geführten Kolben (31). Der Zylinder (11) begrenzt einen Zylinderinnenraum (17). Der Kolben (31) und ein in den Figuren 1 und 2 untenliegender Zylinderboden (13) begrenzen innerhalb des Zylinderinnenraums (17) einen Verdrängungsraum (22), in dem die Injektionslösung (8) gelagert ist.

Der Zylinder (11) hat im Ausführungsbeispiel sowohl eine zylindrische Mantelfläche (18) als auch eine zylindrisch ausgebildete Innenwandung (19). Gegebenenfalls können die Mantelfläche (18) und/oder die Innenwandung (19) auch konisch ausgebildet sein. Der in den Figuren 1 und 2 obenliegende Zylinderkopf (12) ist offen und ohne Abdichtung ausgeführt. Der Zylinderboden (13) des Zylinders (11) ist im Ausführungsbeispiel weitgehend eben ausgebildet. Im abgerundeten Randbereich geht der Zylinderboden (13) stetig in die Mantelfläche (18) des Zylinders (11) über. Die außenliegende Begrenzungsfläche (14) des Zylinderbodens (13) bildet eine Auflagefläche (14) der Zylinder-Kolben-Einheit (10).

Im Zylinderboden (13) sind in den Schnittdarstellungen der Figuren 1 und 2 sechs Durchbrüche (15) dargestellt. Diese verbinden den Zylinderinnenraum (17) mit der Umgebung (1). Der einzelne Durchbruch (15) ist z.B. zylindrisch ausgebildet. Er ist normal zur Auflagefläche (14) ausgerichtet. Beispielsweise haben alle Durchbrüche (15) die gleiche Querschnittsfläche. Der einzelne Durchbruch (15) kann sowohl an seiner dem Zylinderinnenraum (17) zugewandten Seite als auch an seiner der Umgebung (1) zugewandten Seite Anschrägungen und/oder Fasen aufweisen. Im Zylinderboden (13) kann zumindest ein Durchbruch (15) angeordnet sein.

In einer Draufsicht des Zylinderbodens (13) können die Durchbrüche (15) kreisförmig auf einem oder auf mehreren Teilkreisen, spiralförmig, rechteckförmig, etc. angeordnet sein. Hierbei brauchen z.B. die Teilkreise nicht konzentrisch zueinander angeordnet zu sein.

Der Kolben (31) ist beispielsweise scheibenförmig ausgebildet. Er trägt an seiner Mantelfläche (32) z.B. zwei zueinander versetzt angeordnete Kolbendichtelemente (33, 34), mit denen der Kolben (31) an der Zylinderinnenwandung (19) anliegt. Die Kolbendichtelemente (33, 34) sind z.B. O-Ringe mit kreisförmigem Querschnitt. Auch eine andere Ausbildung der Kolbendichtelemente (33, 34) ist denkbar. Beispielsweise können sie unterschiedlich ausgebildet sein.

Der stangenlose Kolben (31) hat im Ausführungsbeispiel eine normal zur Mittenlängsachse (21) der Zylinder-Kolben-Einheit (10) ausgerichtete Kolbenoberseite (35) und eine normal zu dieser Mittenlängsachse (21) orientierte Kolbenstirnseite (36). Die Injektionsvorrichtung umfasst beispielsweise eine Antriebsvorrichtung, mittels der der Kolben (31) in einer in Richtung des Zylinderbodens (13) orientierten Kolbenhubrichtung (37) belastbar ist. Diese Antriebsvorrichtung kann abschaltbar ausgeführt sein, sodass der Kolben (31) entlastbar ist.

In den Durchbrüchen (15) sind Kanülen (50) angeordnet. Beispielsweise kann in jedem Durchbruch (15) eine Hohlnadel (50) sitzen. Es ist auch denkbar, dass die Anzahl der Kanülen (50) kleiner ist als die Anzahl der Durchbrüche (15). Beispielsweise kann die Zylinder-Kolben-Einheit (10) zumindest eine Hohlnadel (50) aufweisen. Diejenigen Durchbrüche (15), in denen keine Kanüle (50) geführt ist, können z.B. mittels eines Verschlusstopfens verschlossen sein. Jede Kanüle (50) sitzt in diesem Ausführungsbeispiel mittels eines die Kanüle (50) umgreifenden Elastomerelements (57) abgedichtet in einem Durchbruch (15). Die einzelne Kanüle (50) ist in dem einzelnen Durchbruch (15) längsverschieblich geführt. Im Ausführungsbeispiel ist die einzelne Kanüle (50) als Hohlnadel mit einer Spitze (52) ausgebildet. Der in Längsrichtung (53) orientierte Kanal (54) dieser Hohlnadel (50) hat über seine Länge eine konstante Innenquerschnittsfläche. Im Ausführungsbeispiel haben alle Kanülen (50) die gleiche Länge. Die einzelne Kanüle (50) hat beispielsweise einen Außendurchmesser von 0,4 Millimetern.

Alle Kanülen (50) sind in einem Vorschubkolben (61) befestigt. Hierbei ist beispielsweise jeweils das in den Figuren 1 und 2 obere Ende der einzelnen Kanüle (50) im Vorschubkolben (61) fixiert, beispielsweise ist es dort verklebt. Die einzelne Kanüle (50) durchdringt den Vorschubkolben (61), sodass der Kanal (54) der Kanüle (50) den Verdrängungsraum (22) mit der Umgebung (1) verbindet. In einer Draufsicht entspricht die Anordnung der Kanülen (50) im Vorschubkolben (61) der Anordnung der Kanülen (50) in den Durchbrüchen (15).

Der Vorschubkolben (61) ist im Ausführungsbeispiel scheibenartig ausgebildet. Er hat z.B. einen zentralen Stempel (78), der in Richtung der Kolbenstirnseite (36) orientiert ist. Der Stempel (78) ist von einem Tragring (79) umgeben, in dem die Kanülen (50) befestigt sind. Die Höhe des Tragrings (79) in der Kolbenhubrichtung (37) beträgt im Ausführungsbeispiel die Hälfte der Höhe des Vorschubkolbens (61).

Zwischen dem Zylinderboden (13) und dem Vorschubkolben (61) ist ein Rückstellelement (67) angeordnet. Im Ausführungsbeispiel ist dies ein Energiespeicher (67) in der Bauform einer Druckfeder (68). Sie ist beispielsweise sowohl am Zylinderboden (13) als auch am Vorschubkolben (61) befestigt. Beispielsweise umgibt der Energiespeicher (67) einzelne Kanülen (50).

In der in der Figur 1 dargestellten Ausgangslage (81) des Vorschubkolbens (61) liegen die Kanülen (50) innerhalb der Hüllfläche der Zylinder-Kolben-Einheit (10). Sie ragen nicht aus der Auflagefläche (14) des Zylinders (11) heraus. Die Summe der Innenquerschnittsflächen der Kanülen (50) ist kleiner als die Querschnittsfläche des die Kanülen (50) tragenden Vorschubkolbens (61). Im Ausführungsbeispiel der Figuren 1 und 2 ist diese Querschnittsfläche die in der Ebene der Nadelbefestigung (55) liegende Fläche des Vorschubkolbens (61). Hierbei ist die Kreisfläche um die Innenquerschnittsflächen der Kanülen (50) reduziert.

Für den Einsatz der Zylinder-Kolben-Einheit (10) wird der Verdrängungsraum (22) mittels einer Injektionslösung (8) befüllt. Das Volumen der Injektionslösung (8) beträgt beispielsweise zwischen 0,1 Millilitern und 3 Millilitern. Die Viskosität der Injektionslösung (8) beträgt beispielsweise 100 Millipascalsekunden.

Nach dem Einsetzen des Kolbens (31) befindet sich dieser in der zurückgefahrenen Position. Das Rückstellelement (67) ist entlastet und hält den Vorschubkolben (61) mit den Kanülen (50) in der Ausgangslage (81). Unmittelbar vor der Anwendung wird beispielsweise ein Originalitätsverschluß entfernt.

Zum Einsatz wird die Zylinder-Kolben-Einheit (10) mit der Auflagefläche (14) auf die Haut (3) eines Patienten aufgesetzt. Die Auflagefläche (14) spannt hierbei die Haut (3). Der Patient spürt nur den Anpressdruck der Zylinder-Kolben-Einheit (10). Mit der Betätigung der Antriebseinheit der Injektionsvorrichtung wird der Kolben (31) in der Kolbenhubrichtung (37), also in den Darstellungen der Figuren 1 und 2 nach unten, belastet. Die Vorschubkraft des in der Kolbenhubrichtung (37) verfahrenden Kolbens (31) erhöht den Innendruck im Verdrängungsraum (22). Dieser Innendruck wirkt auf den Vorschubkolben (61), der entgegen der Rückstellkraft des Energiespeichers (67) in Richtung des Zylinderbodens (13) verschoben wird. Die Kanülen (50) werden aus der Hüllfläche des Zylinderbodens (13) herausgeschoben und dringen in die Haut (3) des Patienten ein. Der Überstand der Kanülen (50) aus dem Zylinderboden (13) beträgt nach dem Ausschieben zwischen 0,6 Millimetern und 1,5 Millimetern. Die Eindringtiefe der Kanülen (50) in die Haut (3) ist damit beispielsweise kürzer als der Abstand der Nervenbahnen zur Oberfläche der Haut (3). Der Patient verspürt somit beim Eindringen der Nadeln keinen oder nur einen geringfügigen Schmerz.

Mit zunehmendem Hub des Kolbens (31) wird Injektionslösung (8) entlang des Tragrings (79) in die Kanülen (50) verdrängt. Durch die Kanäle (54) hindurch wird die Injektionslösung (8) in die Haut (3) gepresst. Sie gelangt hierbei in die Hautschichten (5, 6) unterhalb der Hornhaut (4).

Der Hub des Vorschubkolbens (61) ist beispielsweise durch die Kompression des Energiespeichers (67) begrenzt. Dies ist in der Figur 2 dargestellt. Der Vorschubkolben (61) liegt in seiner vorderen Endlage (82). Die Kanülen (50) sind ausgefahren.

Beim weiteren Verfahren des Kolbens (31) wird weitere Injektionslösung (8) aus dem Verdrängungsraum (22) in die Haut (3) des Patienten gefördert. Beispielsweise ist die Vorschubkraft des Kolbens (31), vermindert um das Verhältnis der Summe der Querschnittsfläche aller Kanülen (50) zur Querschnittsfläche des Kolbens (31) größer als die Rückstellkraft des Energiespeichers (67). Beim weiteren Vorschub des Kolbens (31) verbleibt der Vorschubkolben (61) in der ausgefahrenen Endlage (82). Die Kanülen (50) verbleiben in ihrer Lage in der Haut (3) des Patienten. Die Injektion ist beispielsweise beendet, wenn die Stirnfläche (36) des Kolbens (31) am Stempel (78) des in der ausgefahrenen Endlage (82) stehenden Vorschubkolbens (61) anliegt.

Nach dem Beenden der Injektion wird beispielsweise der Kolben (31) entlastet. Nach dem Entkoppeln der Antriebseinheit vom Kolben (31) wirkt die Rückstellkraft des Energiespeichers (67) auf den Vorschubkolben (61). Der Vorschubkolben (61) wird in die Richtung seiner in der Figur 1 dargestellten Ausgangslage (81) zurück versetzt. Hierbei kann der Vorschubkolben (61) den Kolben (31) verschieben. Gleichzeitig zieht der Vorschubkolben (61) die Kanülen (50) aus der Haut (3) des Patienten heraus. Die Zylinder-Kolben-Einheit (10) kann nun wieder abgenommen werden.

Gegebenenfalls kann die Zylinder-Kolben-Einheit (10) auch ohne Rückstellelement (67) ausgebildet sein.

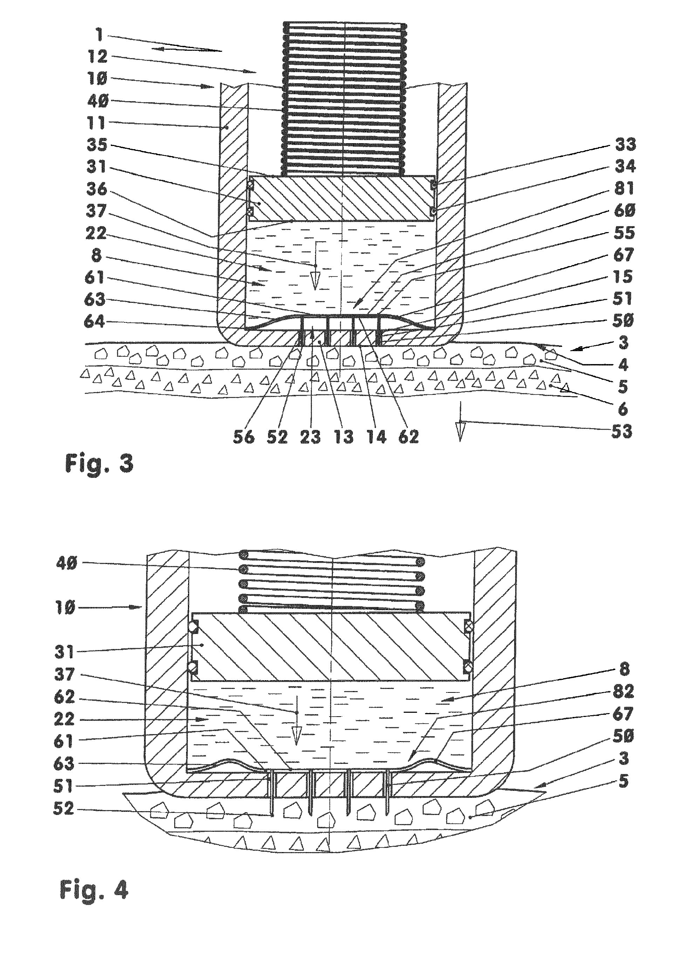

In den Figuren 3 und 4 ist eine Variante einer Zylinder-Kolben-Einheit (10) einer Injektionsvorrichtung dargestellt. Auch in diesem Ausführungsbeispiel umfasst die Zylinder-Kolben-Einheit (10) einen Zylinder (11), einen in diesem geführten Kolben (31) und einen Vorschubkolben (61).

Der Kolben (31) ist so aufgebaut, wie im Zusammenhang mit dem ersten Ausführungsbeispiel beschrieben. Auf der Kolbenoberseite (35) stützt sich beispielsweise ein Kraftspeicher (40) z.B. eine Druckfeder (40) ab. Diese ist z.B. in der gespannten Stellung verriegelbar. Das - hier nicht dargestellte - andere Ende dieser Auslösefeder (40) ist in einem Gehäuse abgestützt, das den Zylinder (11) der Zylinder-Kolben-Einheit (10) trägt. Anstatt mittels einer Druckfeder (40) kann der Kolben (31) auch mittels eines anderen Auslöselements belastbar sein.

Der Zylinder (11) hat auch in diesem Ausführungsbeispiel einen Zylinderboden (13) mit Durchbrüchen (15), in denen Kanüle (50) sitzen. Jede einzelne Kanüle (50) ist hier mit einer Spielpassung (51) in einem Durchbruch (15) längsverschieblich geführt. Die einzelne Kanüle (50) hat eine z.B. außermittige Spitze (52). Die Länge der Kanülen (50) entspricht beispielsweise der zweifachen Dicke des Zylinderbodens (13).

Die im Vorschubkolben (61) befestigten, z.B. verklebten Kanülen (50) durchdringen den Vorschubkolben (61). Der einzelne, eine Kanüle (50) durchdringende Kanal (54) verbindet den zwischen dem Kolben (31) und dem Vorschubkolben (61) liegenden Verdrängungsraum (22) der Zylinder-Kolben-Einheit (10) mit der Umgebung (1). In einer Draufsicht entspricht die Anordnung der Kanülen (50) im Vorschubkolben (61) der Anordnung der Kanülen (50) in den Durchbrüchen (15) und im Zylinderboden (13). In dem in den Figuren 3 und 4 dargestellten Ausführungsbeispiel trägt der Vorschubkolben (61) sämtliche Kanülen (50).

Der z.B. plattenartig ausgebildete Vorschubkolben (61) wird in dem in den Figuren 3 und 4 dargestellten Ausführungsbeispiel durch den zentralen Bereich (62) einer tellerartig ausgebildeten Membran (60) gebildet. Die Membran (60) umfasst einen koaxial zum zentralen Bereich (62) angeordneten Übergangsbereich (63) und einen hierzu ebenfalls koaxialen Randbereich (64). Die Membran (60) kann aus einem homogenen Werkstoff, z.B. einem austenitischen Stahl, einem thermoplastischen Kunststoff, etc. hergestellt sein. Bei einem Kuststoff kann dies beispielsweise Cycloolefin-Copolymere (COC), Polytetraflourethylen (PTFE), etc. sein. Auch die Herstellung aus einem Verbundwerkstoff ist denkbar. Bei einer aus einem Verbundwerkstoff hergestellten Membran (60) hat beispielsweise der zentrale Bereich (62) einen höheren Elastizitätsmodul als der Übergangsbereich (63). Der Randbereich (64) kann den Elastizitätsmodul des zentralen Bereichs (62) oder des Übergangsbereichs (63) aufweisen.

Zumindest der Übergangsbereich (63) ist elastisch verformbar. In der in der Figur 3 dargestellten unverformten Lage ist der Übergangsbereich (63) kegelstumpfförmig ausgebildet, wobei die gedachte Spitze in Richtung des Kolbens (31) zeigt. Der Randbereich (64) der Membran (60) ist am Zylinderboden (13) befestigt. Beispielsweise ist er dort flüssigkeitsdicht verklebt. Der Vorschubkolben (61) ist damit an den Zylinder (11) angedichtet.

In der in der Figur 3 dargestellten Ausgangslage (81) liegen die Kanülen (50) innerhalb der Hüllfläche der Zylinder-Kolben-Einheit (10). Sie ragen nicht aus der Auflagefläche (14) des Zylinders (11) heraus. Die Summe der Innenquerschnittsflächen der Kanülen (50) ist kleiner als die Querschnittsfläche des die Kanülen (50) tragenden Vorschubkolbens (61). Im Ausführungsbeispiel der Figuren 3 und 4 ist diese Querschnittsfläche die in der Ebene der Nadelbefestigung (55) liegende Fläche des zentralen Bereichs (62). Hierbei ist die Kreisfläche um die Innenquerschnittsflächen der Kanülen (50) reduziert.

Die Einsatzvorbereitung dieser Zylinder-Kolben-Einheit (10) erfolgt wie bei der im ersten Ausführungsbeispiel beschriebenen Zylinder-Kolben-Einheit (10).

Beispielsweise nach dem Auslösen der Zylinder-Kolben-Einheit (10) belastet die sich entspannende Druckfeder (40) den Kolben (31). Die durch die Druckfeder (40) erzeugte Vorschubkraft des Kolbens (31) erhöht den Innendruck im Verdrängungsraum (22). Dieser Innendruck wirkt auf den Vorschubkolben (61), der entgegen der Rückstellkraft des elastisch verformbaren Übergangsbereichs (63) in Richtung des Zylinderbodens (13) verschoben wird. Der Übergangsbereich (63) bildet hierbei einen wiederholbar be- und entladbaren Energiespeicher (67).

Beim Hub des Vorschubkolbens (61) in Richtung des Zylinderbodens (13) wird Luft aus dem zwischen dem Vorschubkolben (61) und dem Zylinderboden (13) angeordneten Hubraum (23) entlang der Passungsspalte (56) zwischen den Kanülen (50) und den Durchbrüchen (15) in die Umgebung (1) verdrängt. Gegebenenfalls erfolgt die Verdrängung auch durch Durchbrüche (15), in denen keine Kanüle (50) sitzt. Zumindest entlang der Passungsspalte (56) ist der Hubraum (23) mit der Umgebung (1) verbunden.

Der Vorschubkolben (61) verschiebt die Kanülen (50) in den Durchbrüchen (15). Die Kanülen (50) treten aus der Hüllfläche der Auflagefläche (14) aus. Sie dringen in die Haut (3) des Patienten ein, wobei sie die Hormhaut (4) durchstoßen. Der Patient empfindet hierbei nur einen geringen Schmerz. Der Hub des Vorschubkolbens (61) und der Kanülen (50) ist beispielsweise durch die Anlage des Vorschubkolbens (61) am Zylinderboden (13) begrenzt, vgl. Figur 4. Die Kanülen (50) stehen in dieser ausgefahrenen Endlage beispielsweise mit einer Länge von maximal 1,5 Millimetern aus dem Zylinderboden (13) heraus. Sie ragen beispielsweise in die Hautschicht (5) unterhalb der Hornhaut (4).

Die Injektionslösung (8), die weiterhin auf den Vorschubkolben (61) drückt, durchdringt die Kanülen (50) und wird in die Haut (3) gepresst. Hierbei ist die Vorschubkraft des Kolbens (31), vermindert um das Verhältnis der Summe der Querschnittsfläche aller Kanülen (50) zur Querschnittsfläche des Kolbens (31) größer als die Rückstellkraft des elastisch verformbaren Elements (67). Beim weiteren Vorschub des Kolbens (31) verbleibt der Vorschubkolben (61) in der ausgefahrenen Endlage (82). Die Kanülen (50) verbleiben in ihrer Lage in der Haut (3) des Patienten.

Nach dem Beenden der Injektion wird beispielsweise der Kolben (31) entlastet. Hierfür kann z.B. die am Kolben (31) anliegende Druckfeder (40) entfernt oder gespannt werden. Bei einem manuell betätigten Kolbenvorschub wird dieser beispielsweise losgelassen. Nach dem Entkoppeln des Kraftspeichers (40) vom Kolben (31) wirkt die Rückstellkraft des elastisch verformbaren Elements (67) auf den Vorschubkolben (61). Der Vorschubkolben (61) wird in die Richtung seiner in der Figur 3 dargestellten Ausgangslage (81) zurückversetzt. Hierbei kann der Vorschubkolben (61) den

Kolben (31) in Richtung seiner Ausgangslage vor Beginn der Injektion verschieben. Gleichzeitig zieht der Vorschubkolben (61) die Kanülen (50) aus der Haut (3) des Patienten heraus. Die Zylinder-Kolben-Einheit (10) kann nun wieder abgenommen werden. Gegebenenfalls kann auch diese Zylinder-Kolben-Einheit (10) ohne Rückstellelement (67) ausgebildet sein.

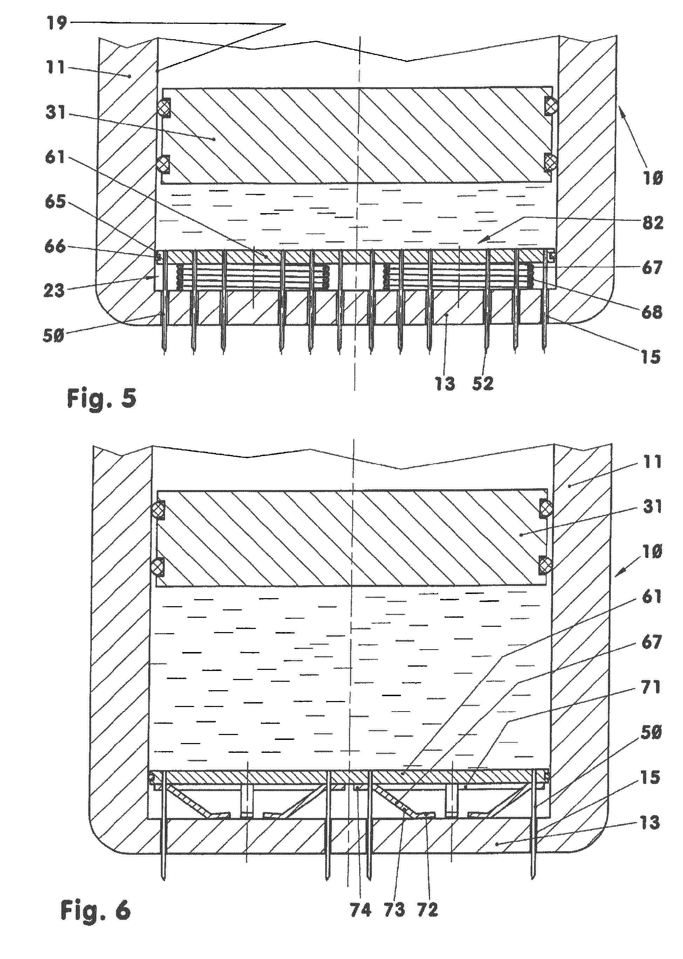

Die Figur 5 zeigt eine Zylinder-Kolben-Einheit (10), deren Rückstellelemente (67) als Druckfedern (68) ausgebildet sind. Anstatt der hier dargestellten zwei Rückstellelemente (67) ist auch eine Ausführung mit einer einzigen, mit drei, vier oder mehr Druckfedern (68) denkbar. Die Zylinder-Kolben-Einheit (10) kann auch ohne Rückstellelement (67) ausgeführt sein. Der Vorschubkolben (61) trägt an seiner Umfangsfläche (65) ein Dichtelement (66). Dies ist beispielsweise ein O-Ring (66), mit dem der Vorschubkolben (61) an der Zylinderinnenwandung (19) anliegt.

Der Zylinder (11) und der Kolben (31) sind in diesem Beispiel so ausgebildet wie im ersten Ausführungsbeispiel. Die Vorschubkraft auf den Kolben (31) kann auch in diesem Ausführungsbeispiel mittels eines Kraftspeichers oder manuell aufgebracht werden.

Der Vorschubkolben (61) trägt die Kanülen (50), die den Hubraum (23) und den Zylinderboden (13) durchdringen. In der in der Figur 5 dargestellten Hubendlage (82) des Vorschubkolbens (61) stehen die Kanülen (50) in ihrer ausgefahrenen Endlage. Die Summe der Innenquerschnittsflächen der verschiebbar in den Durchbrüchen (15) gelagerten Kanülen (50) ist kleiner als die Querschnittsfläche des Vorschubkolbens (61).

Die Funktion der in der Figur 5 dargestellten Vorrichtung entspricht der Funktion der in den Figuren 1 - 4 dargestellten Zylinder-Kolben-Einheiten (10). Die z.B. mittels eines Kraftspeichers erzeugte Vorschubkraft verschiebt den Kolben (31) in Richtung des Zylinderbodens (13). Hierbei wird der Vorschubkolben (61) ebenfalls in Richtung des Zylinderbodens (13) bewegt und die Injektionslösung (8) durch die Kanülen (50) hindurch ausgepresst. Gleichzeitig werden die Luft aus dem Hubraum (23) verdrängt und die Rückstellelemente (67) komprimiert.

Nach dem Beenden der Injektion können die sich entspannenden Rückstellelemente (67) einen Rückhub des Vorschubkolbens (61) und damit ein Herausziehen der Kanülen (50) bewirken. Der Vorschubkolben (61) kann hierbei den Kolben (31) zurückschieben.

In der Figur 6 ist eine Zylinder-Kolben-Einheit (10) mit einem Rückstellelement (67) in der Bauart einer Biegefeder (71) dargestellt. Der Zylinder (11), der Kolben (31) und der Vorschubkolben (61) sind so ausgebildet, wie im Zusammenhang mit dem Ausführungsbeispiel der Figur 5 beschrieben. Die beispielsweise aus einem austenitschen, rost- und säurebeständigen Stahl hergestellte Biegefeder (71) hat einen Abtützbereich (72), einen Übergangsbereich (73) und einen Auflagebereich (74).

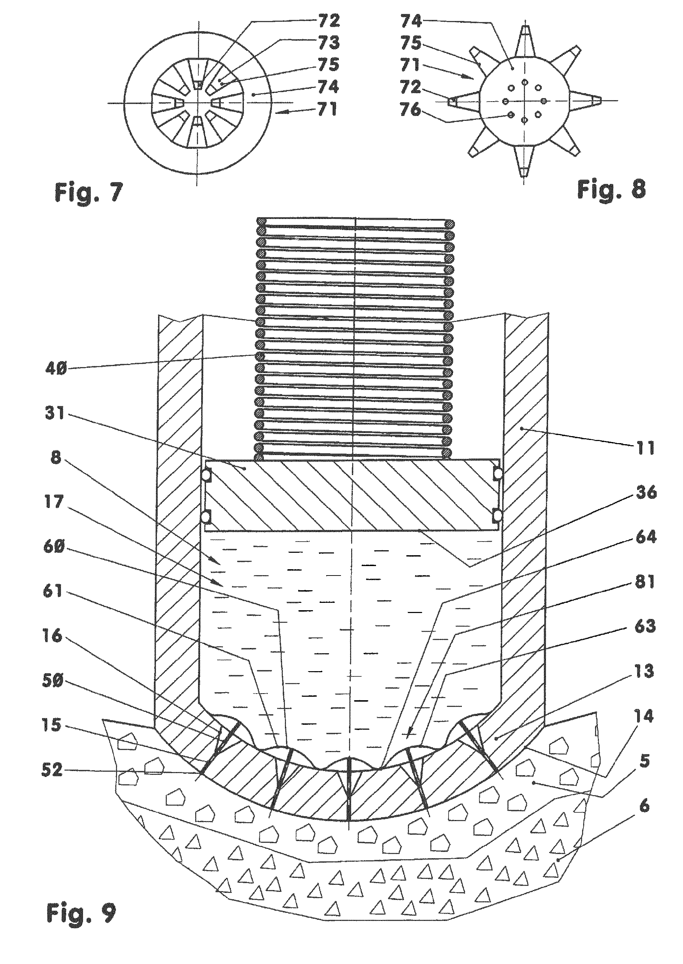

Die Figur 7 zeigt eine Draufsicht einer derartigen Biegefeder (71). Der außenliegende Auflagebereich (74) ist ringförmig ausgebildet. Er stützt den Vorschubkolben (61) ab. Die am Innkreis angeordneten, abgewinkelten zungenartigen Stege (75) bilden den Übergangsbereich (73) und den Abstützbereich (72). Der Abstützbereich (72) liegt in diesem Ausführungsbeispiel auf dem Zylinderboden (13) auf. Selbstverständlich ist es auch denkbar, den Auflagebereich (74) auf den Zylinderboden (13) aufzulegen, sodass der Abstützbereich (72) den Vorschubkolben (61) abstützt.

Beim Vorschub des Kolbens (31) wird auch in diesem Ausführungsbeispiel der Vorschubkolben (61) in Richtung des Zylinderbodens (13) verschoben. Hierbei werden die in den Durchbrüchen (15) verschiebbar gelagerten Kanülen (50) aus dem Zylinder (11) ausgeschoben. Die Biegefeder (71) wird komprimiert, wobei die Abstützbereiche (72) nach innen wandern. Nach der Entlastung des Kolbens (31) können die Biegefedern (71) wieder ihre urprüngliche Gestalt annehmen.

Die Figur 8 zeigt eine Variante der Biegefeder (71) aus der Figur 6. Sie hat einen innenliegenden Auflagebereich (74). Die Stege (75), die den Übergangsbereich (73) und den Abstützbereich (72) bilden, stehen strahlenförmig nach außen ab. Eine Zylinder-Kolben-Einheit (10) mit einem derartigen Rückstellelement (67) funktioniert wie die im Zusammenhang mit den Figuren 6 und 7 beschriebene Vorrichtung.

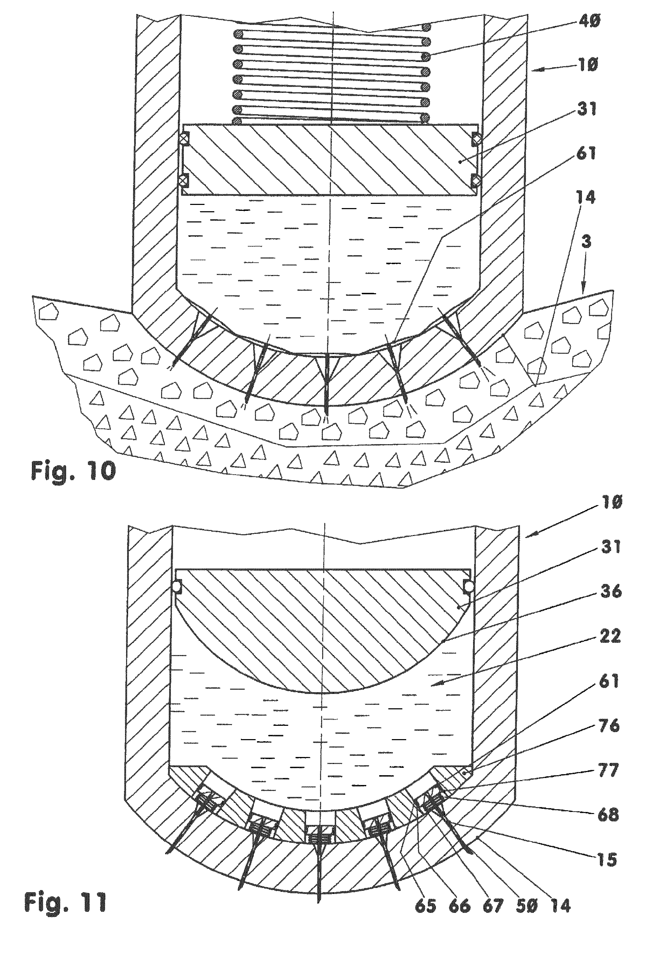

In den Figuren 9 und 10 ist eine Zylinder-Kolben-Einheit (10) dargestellt, deren Zylinder (11) einen Zylinderboden (13) mit einer konvexen Auflagefläche (14) hat. Im Zylinderboden (13) sind in diesem Ausführungsbeispiel radial orientierte Durchbrüche (15) angeordnet. Es ist aber auch denkbar, einzelne oder alle Duchbrüche (15) in nicht radialer Richtung anzuordnen. Die in den Figuren 9 und 10 dargestellten Durchbrüche (15) sind an der in den Zylinderinnenraum (17) mündenden Seite angeschrägt. Diese kegelförmige Anschrägung (16) geht jeweils in den ansonsten zylindrisch ausgebildeten Durchbruch (15) über. Die Durchbrüche (15) können auch in diesem Ausführungsbeispiel ohne Anschrägung (16) ausgebildet sein. Beispielsweise in jedem Durchbruch (15) ist eine Kanüle (50) mit einer Spielpassung (51) in Längsrichtung des Durchbruchs (15) verschiebbar gelagert.

Jede Kanüle (50) ist in einem Vorschubkolben (61) befestigt. Sie durchdringt den Vorschubkolben (61). Ihre Spitze (52) ist in der Darstellung der Figur 9 innerhalb der Hüllfläche der Zylinder-Kolben-Einheit (10) gelagert. In diesem Ausführungsbeispiel entspricht die Anzahl der Kanülen (50) der Anzahl der Vorschubkolben (61). Die Innenquerschnittsfläche jeder Kanüle (50) ist kleiner als die wirksame Querschnittsfläche des zugehörigen Vorschubkolbens (61). Die wirksame Querschnittsfläche ist beispielsweise diejenige Fläche des Vorschubkolbens (61), deren Lage oder deren Gestalt sich bei einer Druckbeaufschlagung ändert. Jeder Vorschubkolben (61) ist Teil einer Membran (60). Die einzelne Membran (60) hat einen elastisch verformbaren Übergangsbereich (63) und einen Randbereich (64), der am Zylinder (11) befestigt ist. Beispielsweise sind die Randbereiche (64) aller Membrane flüssigkeitsdicht am Zylinderboden (13) verklebt.

Der Kolben (31) und der Kraftspeicher (40) sind in diesem Ausführungsbeispiel so ausgebildet, wie im Zusammenhang mit dem ersten Ausführungsbeispiel beschrieben. Die dem Verdrängungsraum (22) zugewandte Stirnseite (36) des Kolbens (31) kann auch konvex oder kegelförmig ausgebildet sein.

Um die Injektionsflüssigkeit (8) in die Haut (3) des Patienten einzubringen, wird die Zylinder-Kolben-Einheit (10) mit der Anlagefläche (14) auf die Haut (3) des Patienten aufgesetzt. Die Zylinder-Kolben-Einheit (10) drückt die Haut (3) ein. Sie spannt diese, sodass sich die Haut (3) an die Auflagefläche (14) anlegt.

Bei Belastung des Kolbens (31) mittels des Kraftspeichers (40) wird der statische Druck der Injektionsflüssigkeit (8) erhöht. Die Injektionsflüssigkeit (8) drückt auf die Vorschubkolben (61) und auf die mit diesen verbundenen Kanülen (50). Die Kanülen (50) werden nach außen gedrückt und dringen in die Haut (3) des Patienten ein. Die Injektionsflüssigkeit (8) wird in die Haut (3) des Patienten eingebracht, vgl. Figur 10.

Nach Beendigung der Injektion wird beispielsweise der Kolben (31) zurückgezogen. Die einzelnen Rückstellelemente (67) verformen sich zurück in die Ausgangsgestalt. Hierbei werden die Vorschubkolben (61) in die in der Figur 9 dargestellte Ausgangslage (81) zurückverschoben. Die Vorschubkolben (61) ziehen hierbei die Kanülen (50) in die eingefahrene Ausgangslage zurück. Die Kanülen (50) stehen jetzt wieder innerhalb der Hüllfläche der Zylinder-Kolben-Einheit (10).

Die Figur 11 zeigt eine Variante einer Zylinder-Kolben-Einheit (10) mit einer konvexen Auflagefläche (14). Der Kolben (31) hat beispielsweise eine dem Verdrängungsraum (22) zugewandte konvex ausgebildete Kolbenstirnseite (36). Der Radius der Kolbenstirnseite (36) entspricht beispielsweise dem Innenradius eines auf dem Zylinderboden (13) angeordneten Zylindereinsatzes (76). Der Zylindereinsatz (76) ist z.B. mit dem Zylinderboden (13) stoffschlüssig verbunden. Beispielsweise ist er mit diesem flüssigkeitsdicht verklebt. Der Zylindereinsatz (76) kann aber auch in den Zylinderboden (13) integriert sein.

Die Durchbrüche (15) des Zylinderbodens (13) fluchten mit im Zylindereinsatz (76) angeordneten Zylindertöpfen (77). Jede Kanüle (50) ist auch in diesem Ausführungsbeispiel in einem Vorschubkolben (61) befestigt und durchdringt diesen. Der einzelne Vorschubkolben (61) sitzt verschiebbar in einem Zylindertopf (77). Er hat an seiner Umfangsfläche (65) ein Kolbendichtelement (66), z.B. einen O-Ring, das den Vorschubkolben (61) zum Zylindertopf (77) und zum Zylinder (11) hin abdichtet.

Im Zylindertopf (77) ist der einzelne Vorschubkolben (61) mittels eines Rückstellelements (67) abgestützt. Das einzelne Rückstellelement (67) ist beispielsweise eine Druckfeder (68), die den Vorschubkolben (61) in Richtung der Ausgangslage belastet.

Beim Vorschub des Kolbens (31) im Zylinder (11) wird auch in diesem Ausführungsbeispiel die Vorschubkraft auf die Injektionsflüssigkeit (8) übertragen. Die Injektionsflüssigkeit (8) verschiebt die Vorschubkolben (61) mit den Kanülen (50) relativ zu den Zylindertöpfen (77). Die Kanülen (50) dringen in die Haut (3) ein. Gleichzeitig wird Injektionsflüssigkeit (8) durch die Kanülen (50) ausgepresst. Die Injektionsflüssigkeit (8) gelangt in die Schichten (5, 6) der Haut (3) unterhalb der Hornhaut (4).

Nach der Beendigung der Injektion verschieben die Rückstellelemente (67) die Vorschubkolben (61) zurück in die Ausgangslage. Die Kanülen (50) werden eingefahren, sodass sie innerhalb der Hüllfläche des Zylinders (11) der Zylinder-Kolben-Einheit (10) liegen. Die Zylinder-Kolben-Einheit (10) kann nun entsorgt werden, ohne dass keine Gefahr einer Verletzung des Bedieners besteht.

Es ist auch denkbar, die verschiedenen genannten Ausführungsformen miteinander zu kombinieren.

### Bezugszeichenliste:

- 1: Umgebung

- 3: Haut
- 4: Hornhaut
- 5: Hautschicht
- 6: Hautschicht

- 8: Injektionslösung

- 10: Zylinder-Kolben-Einheit
- 11: Zylinder
- 12: Zylinderkopf
- 13: Zylinderboden
- 14: Auflagefläche, Begrenzungsfläche von (13)
- 15: Durchbrüche
- 16: Anschrägung
- 17: Zylinderinnenraum
- 18: Mantelfläche
- 19: Innenwandung, Zylinderinnenwandung

- 21: Mittenlängsachse
- 22: Verdrängungsraum
- 23: Hubraum

- 31: Kolben
- 32: Mantelfläche
- 33: Kolbendichtelement
- 34: Kolbendichtelement
- 35: Kolbenoberseite
- 36: Kolbenunterseite, Kolbenstirnseite
- 37: Kolbenhubrichtung

- 40: Kraftspeicher, Druckfeder, Auslösefeder

- 50: Kanüle, Hohlnadel
- 51: Spielpassung
- 52: Spitze
- 53: Längsrichtung
- 54: Kanal
- 55: Nadelbefestigung
- 56: Passungsspalte
- 57: Elastomerelement

- 60: Membran
- 61: Vorschubkolben
- 62: zentraler Bereich
- 63: Übergangsbereich
- 64: Randbereich
- 65: Umfangsfläche
- 66: Dichtelement, O-Ring
- 67: Rückstellelement, Energiespeicher, elastisch verformbares Element
- 68: Druckfedern

- 71: Biegefeder
- 72: Abstützbereich
- 73: Übergangsbereich
- 74: Auflagebereich
- 75: Stege
- 76: Zylindereinsatz
- 77: Zylindertöpfe
- 78: Stempel
- 79: Tragring

- 81: Ausgangslage
- 82: Endlage

## Patentansprüche

1. Zylinder-Kolben-Einheit (10) mit einem einen Zylinderinnenraum (17) begrenzenden Zylinder (11) und mit einem in diesem geführten Kolben (31), wobei der Zylinder (11) einen Zylinderboden (13) mit mindestens einem Durchbruch (15) aufweist,
**dadurch gekennzeichnet,**
- **dass** im Durchbruch (15) eine Kanüle (50) verschiebbar gelagert ist,
- **dass** die Kanüle (50) an einem im Zylinderinnenraum (17) angeordneten, vom Kolben (31) getrennten Vorschubkolben (61) befestigt ist.

2. Zylinder-Kolben-Einheit (10) nach Anspruch 1,
**dadurch gekennzeichnet,**
**dass** der Vorschubkolben (61) von einer Ausgangslage (81), in der die Kanüle (50) innerhalb der Hüllfläche der Zylinder-Kolben-Einheit (10) liegt in eine Endlage (82), in der die Kanüle (50) aus der Hüllfläche der Zylinder-Kolben-Einheit (10) herausragt, verschiebbar ist.

3. Zylinder-Kolben-Einheit (10) nach Anspruch 2,
**dadurch gekennzeichnet,**
**dass** die einzelne Kanüle (50) in der Endlage (82) des Vorschubkolbens (61) mit einer Länge von maximal 1,5 Millimetern aus dem Zylinder (13) herausragt.

4. Zylinder-Kolben-Einheit (10) nach Anspruch 1,
**dadurch gekennzeichnet,**
**dass** die einzelne Kanüle (50) den Vorschubkolben (61) durchdringt.

5. Zylinder-Kolben-Einheit (10) nach Anspruch 1,
**dadurch gekennzeichnet,**
**dass** der Vorschubkolben (61) mittels eines Rückstellelements (67) belastet ist.

6. Zylinder-Kolben-Einheit (10) nach Anspruch 5,
**dadurch gekennzeichnet,**
**dass** das Rückstellelement (67) ein elastisch verformbares Element ist.

7. Zylinder-Kolben-Einheit (10) nach Anspruch 1,
**dadurch gekennzeichnet,**
**dass** der Vorschubkolben (61) an den Zylinder (11) angedichtet ist.
